# EUROPEAN PATENT SPECIFICATION

(11) **EP 3 615 042 B1**
(45) Date of publication and mention of the grant of the patent: **27.07.2022**
(21) Application number: 18722244.3
(22) Date of filing: 24.04.2018
(51) Int. Cl.: A61K 33/14, A61K 33/42, A61K 33/02, A61K 31/14, A61K 31/198, A61K 31/205, A61K 31/6615, A61K 45/06, A23K 20/24, A23K 20/26, A61K 9/00, A61P 15/06, A61P 43/00, A61K 33/06, A23K 20/00, A23K 20/10, A23K 50/30

(54) **COMPOSITION CONTAINING A CHLORIDE SALT FOR USE IN PREGNANT ANIMALS**
ZUSAMMENSETZUNG DIE EIN CHLORIDSALZ ENTHÄLT ZUR VERWENDUNG BEI TRÄCHTIGEN TIEREN
COMPOSITION CONTENANT UN SEL DE CHLORURE DESTINÉE À ÊTRE UTILISÉE CHEZ DES ANIMAUX EN GESTATION

(30) Priority: 25.04.2017 NL 2018777
(43) Date of publication of application: 04.03.2020
(73) Proprietor: Nutreco IP Assets B.V., 5831 JN Boxmeer (NL)
(72) Inventor: LANGENDIJK, Pieter, 3800 AG Amersfoort (NL)
(74) Representative: EP&C
(86) International application number: PCT/NL2018/050261
(87) International publication number: WO 2018/199749

(56) References cited:
- EP-A1- 1 731 183
- WO-A1-98/11866
- US-A1- 2013 022 733
- TOSHIO NAKATSUKA ET AL: "Effects of sodium bicarbonate and ammonium chloride on the incidence of furosemide-induced fetal skeletal anomaly, wavy rib, in rats", TERATOLOGY., vol. 48, no. 2, 1 August 1993 (1993-08-01) , pages 139-147, XP055438667, US ISSN: 0040-3709, DOI: 10.1002/tera.1420480208
- N.D. ELROD; R.M. HARP,; K.G. BRYAN: "Effect of calcium ion supplementation on swine parturition", THE TEXAS JOURNAL OF AGRICULTURE AND NATURAL RESOURCES, vol. 28, 2015, pages 12-17, XP009502621, cited in the application
- HOLLINSHEAD F K ET AL: "Calcium, parathyroid hormone, oxytocin and pH profiles in the whelping bitch", THERIOGENOLOGY, LOS ALTOS, CA, US, vol. 73, no. 9, 1 June 2010 (2010-06-01), pages 1276-1283, XP027050786, ISSN: 0093-691X [retrieved on 2010-02-19]
- J. Derouchey ET AL: "Sow Feed Additives on the Market: Are They Worth it?", The Pig Site, 15 March 2009 (2009-03-15), pages 1-8, XP055438720, Retrieved from the Internet: URL:http://www.thepigsite.com/articles/264 0/sow-feed-additives-on-the-market-are-the y-worth-it/ [retrieved on 2018-01-08]
- DATABASE WPI Week 201381 Thomson Scientific, London, GB; AN 2013-T75696 XP002782585, & CN 103 211 100 A (UNIV NORTHEAST AGRIC) 24 July 2013 (2013-07-24)
- S.T. LIU ET AL: "Effects of dietary citric acid on performance, digestibility of calcium and phosphorus, milk composition and immunoglobulin in sows during late gestation and lactation", ANIMAL FEED SCIENCE AND TECHNOLOGY, vol. 191, 1 May 2014 (2014-05-01), pages 67-75, XP55488007, AMSTERDAM, NL ISSN: 0377-8401, DOI: 10.1016/j.anifeedsci.2014.01.017
- S.Y. CHENG ET AL: "Effects of dietary electrolyte balance on the performance, plasma biochemistry parameters and immunoglobulin of sows during late gestation and lactation", ANIMAL FEED SCIENCE AND TECHNOLOGY, vol. 200, 1 February 2015 (2015-02-01), pages 93-101, XP55488011, AMSTERDAM, NL ISSN: 0377-8401, DOI: 10.1016/j.anifeedsci.2014.12.011
- DATABASE WPI Week 201422 Thomson Scientific, London, GB; AN 2014-F32508 XP002782586, & CN 103 535 550 A (UNIV NORTHEAST AGRIC) 29 January 2014 (2014-01-29)
- KRUKOWSKI M ET AL: "Acidosis, hypercalcemia, and hyperphosphatemia in rat fetuses near term and effects of maternal acid/base loading.", PROCEEDINGS OF THE SOCIETY FOR EXPERIMENTAL BIOLOGY AND MEDICINE. SOCIETY FOR EXPERIMENTAL BIOLOGY AND MEDICINE (NEW YORK, N.Y.) NOV 1979, vol. 162, no. 2, November 1979 (1979-11), pages 359-364, XP009502597, ISSN: 0037-9727
- Mongin P.: "Recent advances in dietary anion-cation balance: applications in poultry.", Proc. Nutr. Soc., 1 January 1981 (1981-01-01), pages 285-294, XP055835067, Retrieved from the Internet: URL:https://www.cambridge.org/core/journal s/proceedings-of-the-nutrition-society/art icle/recent-advances-in-dietary-anioncatio n-balance-applications-in-poultry/C5733627 64A278489EEAB85CBECE2C25 [retrieved on 2021-08-25]
- Block Elliot: "Revisiting Negative Dietary Cation-Anion Difference Balancing for Prepartum Cows and its Impact on Hypocalcaemia and Performance", , 1 January 2011 (2011-01-01), pages 32-51, XP055833804, Retrieved from the Internet: URL:https://animal.ifas.ufl.edu/apps/dairy media/rns/2011/5block.pdf [retrieved on 2021-08-22]
- DEROUCHEY J M ET AL: "Effects of dietary electrolyte balance on the chemistry of blood and urine in lactating sows and sow litter performance", JOURNAL OF ANIMAL SCIENCE, AMERICAN SOCIETY OF ANIMAL SCIENCE, US, vol. 81, no. 12, 1 December 2003 (2003-12-01), pages 3067-3074, XP009502593, ISSN: 0021-8812, DOI: 10.2527/2003.81123067X

## Description

### GENERAL FIELD OF THE INVENTION

The present invention in general pertains to the field of maximizing the reproductive performance of animals, in particular mammals, further in particular a swine. The invention in particular pertains to maximizing the performance of animals and their offspring by adapting their nutrition. The invention is as defined in the claims.

### BACKGROUND ART

Maximizing the performance of pregnant animals and their offspring has for a long time been a major objective of nutritionists. *Inter alia* feed-processing technologies, amino acid supplementation and increased dietary energy density have been used to address the objective of maximizing this performance. For example, research in dairy cattle, laying hens and swine has indicated positive effects on metabolism, in particular reduced incidence of milk fever, greater egg shell quality and reduction of stillbirth, when the dietary electrolyte balance (dEB) was modified.

Elrod et al. (The Texas Journal of Agriculture and Natural Resources 28:12-17, 2015) discloses an effect of calcium ion supplementation on swine parturition.

DeRouchey et al (The Pig Site, 15 March 2009) discusses sow feed additives on the market.

Database WPI Week 201381 Thomson Scientific London; AN2013-T75696 & CN 103211 100 (UNIV NORTHEAST AGRIC) (24 July 2013) discloses a feed additive for improving immunoglobulin content in sow colostrum.

US20130022733 discloses a calcium preparation and method of production thereof. DeRouchey et al (J. Anim. Sci. 2003. 81:3067-3074) discloses effects of dietary electrolyte balance on the chemistry of blood and urine in lactating sows and sow litter performance.

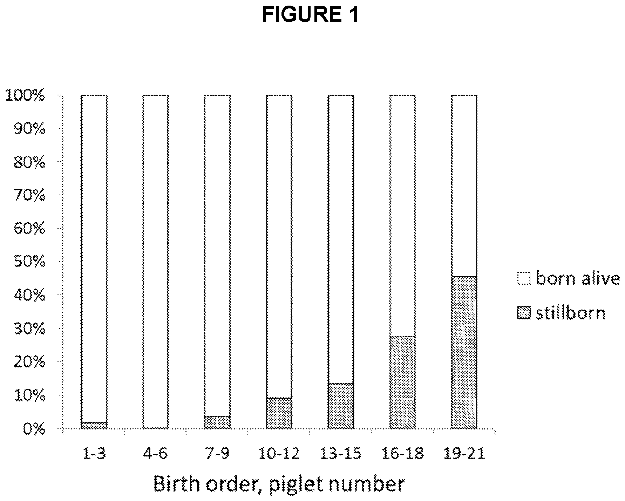

One important aspect of maximizing reproductive performance is optimizing the health of the pregnant animal and its (unborn) offspring during parturition. In particular, it is believed that this may reduce perinatal mortality of the offspring, such as stillbirth (intrapartum mortality) and neonatal mortality. In particular, stillbirth in piglets is mainly caused by oxygen insufficiency, due to repeated episodes of reduced blood perfusion of the placenta, caused by uterine contractions compressing the placenta, and due to stretch and sometimes rupture of the umbilical cord as the foetus travels through the uterus and the birth canal. The oxygen insufficiency leads to increasing blood lactate levels, a decreasing blood pH, and a reduction in extra-cellular fluid base excess in the foetus, a condition referred to as acidosis. When this condition aggravates, this can result in piglet death. Short term oxygen insufficiency is reversible; however, prolonged oxygen insufficiency may lead to irreversible effects and finally, death, and hence it is understandable that stillbirth occurs mainly in piglets born at the end of the birth order (see figure 1). Piglets that are born alive but have suffered from oxygen insufficiency can be permanently affected which is reflected in their behaviour, reduced vigour and reduced performance.

It is commonly believed that one of the main causes for prolonged parturition is a reduced concentration of freely available calcium (ionic Calcium, or iCa) in maternal circulation. Calcium is involved in regulating the force and coordination of uterine contractions, and insufficient iCa in the blood results in poor muscle function, and as a consequence, prolonged parturition. For this reason, calcium supplementation to the diet of pregnant animals is often proposed as a measure to reduce perinatal mortality.

However, the influence of nutrition on maximizing performance, in particular of reproductive performance, has not been conclusively explored yet.

### OBJECT OF THE INVENTION

It is an object of the invention to improve colostrum production of an animal, in particular a sow.

### SUMMARY OF THE INVENTION

The invention is as set out in the claims.

It has been found that it is advantageous to use a blood pH modulator such as a chloride salt for oral administration to maintain in a pregnant animal its blood pH at a physiological level. It appears that by maintaining the pH in the blood of the pregnant animal at its physiological level, perinatal mortality can be reduced, and colostrum uptake of neonates and production of a (lactating) animal can be improved. The basis for this finding is the recognition by applicant that pregnant animals with prolonged parturition, when compared to animals with uncomplicated parturitions, have a supranormal blood pH (for sows typically about 7.50 vs 7.45, *i.e.* in venous blood), and a concomitant lower than normal blood pCO₂, which phenomena are typical for respiratory alkalosis. The latter condition is believed to be caused by an increased respiratory rate in sows approaching parturition. It is believed that this hyperventilation causes increased evacuation of CO₂ from the circulation, causing the Henderson-Hasselbalch equation (H₂O + CO₂ ↑ 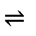 H₂CO₃ 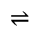 H⁺ + HCO₃⁻ ) to shift to the left, with a drop in [H⁺] and a higher pH as a result. Some sows are apparently able to cope with the changes in acid-base balance, and maintain their blood pH, whereas some sows develop an increased pH. This increase in pH has been found to be directly related to perinatal mortality, in particular to stillbirth and neonatal mortality of the offspring. In particular it has been found that by maintaining the blood pH at its physiological level, the number of stillborn offspring of the pregnant animal and/or the percentage of animals having one or more stillborn offspring can be reduced. Also, colostrum production by the lactating animal and colostrum uptake of the offspring can be improved by applying the blood pH modulator. This may have a positive influence on improving postnatal survival of the offspring, in particular neonatal survival of the offspring.

It is noted that in the art it is described to reduce the pH to a level *below* a physiological level in order to improve reproductive performance. DeRouchey et al. in Swine Research 2005, pp 34-37, shows that lowering the pH in the gastro intestinal tract and urine to below a normal level may be helpful, albeit to a very minor extent, to reduce the number of stillborn animals. In J. Anim. Sci. (2003, 81: 3167-3074), DeRouchey et al. show that a reduction of the blood pH to *below* a physiological level had a positive effect on piglet survivability, although no correlation could be found with litter and sow weights and the number of stillborn piglets. Elrod et al. in The Texas Journal of Agriculture and Natural Resources (2015, 28: 12-17) show that calcium ion supplementation for five days pre-farrowing may lead to a decrease of the urinary pH level *below* the physiological level of about 7.3 to a level as low as 5.65. At the same time, the duration of parturition and the number of stillbirths was also reduced. Elrod drew the conclusion that the data support the positive effect of calcium ion supplementation on swine parturition. It thus appears that the art is aiming mainly to positively influence the availability of iCa in the animal by decreasing the pH to a value *below* a physiological level.

Contrary to what the art teaches, it was presently found that *maintaining* the pH in the blood at a physiological level, a remarkably positive influence on reproduction performance and colostrum production and uptake, in particular a reduction of perinatal mortality, can be obtained.

### BRIEF DESCRIPTION OF THE FIGURES

Figure 1 shows the percentage of piglets born alive or stillborn depending on the birth order. It demonstrates that stillbirth occurs mainly in piglets born at the end of the birth order.
Figure 2 shows the change in blood pH in pregnant sows approaching parturition.
Figure 3 shows the blood pH for pregnant sows receiving a daily cation/anion balance of 270 mEq/d ("low dEB") and 675 mEq/d ("high dEB"), respectively.

### DEFINITIONS

A *blood pH modulator* is a compound, for example a pure chemical substance or a mixture of substances that after digestion modulates the pH of the blood, for example by lowering or raising the pH. Common blood pH modulators are inorganic substances such as minerals that alter the dietary electrolyte balance, and organic substances such as organic acids. Typically, a blood pH modulator is administered as a feed or drinking water additive, but it is noted that a particular feed (as a whole) can also be regarded as a "blood pH modulator", for example by extracting certain components in order to modulate the blood pH.

A *physiological* level of the blood pH of a pregnant animal is the pH which is characteristic of the healthy and normal functioning animal that lives of a normal diet. Healthy means that there is no significant negative effect on the health of the animal itself. Typically, a pregnant animal until a period of at least 5-10 days before parturition has a blood pH at physiological level, in particular until at least 48 hours before parturition. For a pregnant sow, the physiological blood pH of venous blood is typically 7.45 with a standard deviation of 0.03 (within a group of animals).

An animal's *diet* is the habitual nourishment of the animal, including feed (solid and liquid feed) and drinking water.

A *calcium binder* is a compound that prevents calcium ions from being dissolved in water in a concentration of more than 10 mM, in particular more than 1 mM.

A salt is *insoluble* in water if an aqueous solution of the salt contains less than 1 mmol of the salt per liter of water at room temperature.

A *dietary supplement* is a product intended for ingestion, which contains a dietary ingredient intended to add nutritional value to the diet. The product can for example be added to the solid feed of the animals (in which case the supplement is often referred to as a top-dress) or to the drinking water (in which case the supplement is often referred to as a drinking water additive).

*Perinatal mortality* is the total of stillbirths and neonatal deaths.

*Stillbirth* is the phenomenon where offspring is born dead, but has died only shortly before (in particular up to 24-48 hours) or during parturition.

*Neonatal death* is the phenomenon where offspring dies shortly after birth, typically within 10 days after birth.

*Postnatal mortality* refers to deaths occurring after birth but before weaning, and includes neonatal deaths.

*Colostrum* is the milk secreted between parturition and 24 h thereafter.

*Colostrum uptake* is calculated based on the increase in body weight between birth and 24 h thereafter.

### DETAILED DESCRIPTION OF THE DISCLOSURE

The present disclosure describes a chloride salt for use in improving colostrum production of an animal, improving colostrum uptake in offspring of an animal, and improving neonatal/postnatal survival in the offspring of an animal. The invention is as defined in the claims.

The animal is preferably a pregnant animal, such as a non-human pregnant animal, preferably a pregnant farming animal, even more preferably a pregnant monogastric farming animal. According to the invention, the animal is a pregnant sow. As such, the offspring is piglets.

The blood pH modulator may be used to maintain the pH at a physiological level within 0 to 36 hours before parturition and/or during parturition, in particular within 0 to 24 hours before parturition and/or during parturition. It was found, in particular for pregnant swine, that the pH levels typically increase to above a physiological level in the window of 0-36 hours, in particular 0-24 hours before parturition. Ideally, the pH is maintained at a physiological level during this critical period.

In an embodiment the blood pH modulator, i.e. a chloride salt, is added to the animal's diet (which also encompasses enriching the diet in certain components by extracting other ones). This was found to be a convenient way to provide for oral administration. The blood pH modulator, i.e. a chloride salt, may be added to the drinking water or feed of the animal. In particular, the blood pH modulator, i.e. chloride salt, may be added to the drinking water of the animal. The advantage thereof is that pregnant animals, even right before parturition, normally show regular or even increased drinking behaviour, whereas they might decrease or even cease the eating of solid feed. Thus, consumption of the blood pH modulator, i.e. chloride salt, may be continued particularly at the point in time when approaching parturition, which is when the respiratory rate in sows increases, and when the blood pH modulator is most effective.

In yet another embodiment the blood pH modulator, i.e. chloride salt, is administered in a period of 0 to 5 days before parturition, in particular at least in a period of 1 to 5 days before parturition (which does not exclude that the modulator is also administered earlier or later, for example during parturition). It was found that by administering the blood pH modulator during this period, the pH may be kept at its physiological level before and potentially also during parturition.

In still another embodiment the blood pH modulator comprises chloride salt, chosen from ammonium chloride, or calcium chloride or organic chloride salts, such as betaine HCI, lysine HCI or choline chloride. According to the invention, the blood pH modulator is a chloride salt as taught herein. It was shown that by using the blood pH modulator, which is a chloride salt, the pH of the blood can be modulated such that before and during parturition the pH remains at a physiological level.

In an embodiment, the blood pH modulator, i.e. chloride salt, is added to the animal's diet, e.g., by means of addition of the modulator to the drinking water or feed.

In an embodiment, the blood pH modulator, which is a chloride salt, is administered to said animal in such an amount that the electrolyte balance dEB of the diet is in the range of about 0-400 mEq/day, such as in the range of about 25-375 mEq/day, about 50-350 mEq/day, about 75-325 mEq/day, about 100-300 mEq/day, about 125-275 mEq/day, or about 150-250 mEq/day.

The amount of the modulator, i.e. chloride salt that is added to the animal's diet in the form of feed may be such that it allows to obtain an electrolyte balance of 50 to 150 mEq/kg in the total diet. This means that in the total dietary intake the average value of the electrolyte balance may be between 50 and 150 mEq/kg. This may be any value such as 50, 55, 60, 65, 70, 75, 80, 85, 90, 95, 100, 105, 110, 115, 120, 125, 130, 135, 140, 145 and 150 mEq/kg of the total diet. As an example, when administering the blood pH modulating mineral in a top-dress that is dosed at 400 g per day, the value for the top-dress as such may be around -600 mEq/kg. This way, by administering the top-dress, the total daily diet will have a lowered electrolyte balance of about -240 mEq per day (which is about -100 mEq/kg assuming a total intake of 2.4 kg per day). For a calculation of the amount of mineral in mEq, referral is made to the paper by Elliot Block, Arm & Hammer Animal Nutrition, Princeton, NJ, USA, called "Revisiting Negative Dietary Cation-Anion Difference Balancing for Prepartum Cows and its Impact on Hypocalcaemia and Performance" (http://dairy.ifas.ufl.edu/rns/2011/5block.pdf), in particular to page 35 where an example is given for a calculation of a mineral concentrations in mEq.

In a suitable embodiment, the blood pH modulator, i.e. chloride salt, is added to the drinking water of the animal, and the drinking water of the animal comprises between about -5 and -45 mEq/L, such as between about -10 and - 40, between about -15 and - 35, between about -15 and -30, or between about -15 and -25 mEq/L of said blood pH modulator, in particular said chloride salt.

In an embodiment, said blood pH modulator, i.e. chloride salt, is administered in a period of 0 to 5 days before parturition, in particular at least in a period of 1 to 5 days before parturition.

In an embodiment, a calcium binder may additionally be added to the animal's diet, such as to the feed or to the drinking water. For example, the calcium binder may be contained in a composition which is in the form of a premix, a liquid, a powder, granules, a pellet, a dragee, a tablet, a pill, or a capsule.

In a particularly suitable embodiment, the calcium binder may be added to the drinking water of the animal, for the same reason as mentioned above in respect of the blood pH modulator.

Completely contrary to the prior art teaching that it is advantageous to supplement the diet with extra calcium, it was found that the addition of a calcium binder to the diet has a positive effect on perinatal mortality, in particular on a reduction of stillbirth. Without being bound to theory, it is believed that by effectively lowering the amount of available calcium in the animal's diet, the animal's natural capability to release calcium from its mineral reserves (in particular from the bone) might be stimulated. In a particular embodiment, the calcium binder is an anion that forms a water insoluble salt with calcium ions in aqueous solution. Such an ion may for example be a conjugated base ion.

A calcium binder according to the present invention is selected from the group consisting of phosphoric acid, phytic acid, a phytate, a polyphosphate, a tripolyphosphate, a phosphate and a cellulose phosphate.

Calcium binders include any compounds which are capable of binding free calcium in the ruminant gastro-intestinal tract whereby the free calcium cannot be absorbed by the animal. Thus, the natural calcium regulating defence mechanism of the animal is triggered. Such compounds include phosphoric acid, oxalic acid, sodium oxalate, phytic acid, a phytate, a clay mineral including zeolite, sodium diethylene acetic acid, ethylene diaminetetraacetic acid (EDTA), the sodium salts of EDTA Na2EDTA and Na4EDTA, trisodium nitrilotriacetate monohydrate, trisodium ntrioacetate, pentasodium diethylenetriaminepentaacetate, trisodium N-hydroxyethylethylenediaminetriacetate, citric acid, a citrate, a polyphosphate, a tripolyphosphate, a phosphate, a cellulose phosphate, glutamic acid N,N-diacetic acid (GLDA), a sodium salt of GLDA, a potassium salt of GLDA, methylglycine-N,N-diacetic acid (MGDA), a sodium salt of MGDA, a potassium salt of MGDA, ethylenediamine N,N'-disuccinic acid (EDDS), a sodium salt of EDDS, a potassium salt of EDDS, iminodisuccinic acid (IDS), a sodium salt of IDS, or a potassium salt of IDS, or a calcium-free derivative of any such compounds.

The calcium binder may advantageously be phosphoric acid or a phosphate, particularly when the calcium binder is added to drinking water. Phosphoric acid has the interesting property that it does not form a water insoluble salt with calcium ions at low pH (i.e., when in solution prior to administration to an animal, it remains in soluble form, even when formulated with a calcium salt such as calcium chloride), whilst it precipitates with calcium ions at physiological pH (i.e., in the gastrointestinal tract of the animal). The calcium binder is preferably an anion that forms a water insoluble salt with calcium ions at physiological small intestinal pH (e.g., a pH in the range of 6-7.5, preferably 6.5-7.5) but not at acidic pH (e.g., a pH below 6, preferably below 5.5).

The calcium binder may be encapsulated by any appropriate encapsulating material. A compound suitable for encapsulation of calcium binder is a compound selected from the groups consisting of a fat, a non-calcium derivative of a fat such as a soap and a stearate, a protein, a polysaccharide, a cellulose and a derivative of any such compound, a gum, a glycol and gelatine.

The latter teaching of advantageously using an additional calcium binder, provides that the invention is also embodied in the use of a blood pH modulator which is a chloride salt in combination with a calcium binder such as phosphoric acid to supplement a diet of a pregnant animal in a period of 0 to 5 days before parturition and in a dietary supplement comprising in combination a blood pH modulator which is a chloride salt, e.g., calcium chloride, and a calcium binder such as phosphoric acid.

The addition of a calcium binder is particularly advantageous when calcium chloride is used as the chloride salt.

### Compositions and uses thereof

The present disclosure teaches an aqueous composition comprising a chloride salt as taught herein such as calcium chloride and a calcium binder as taught herein such as phosphoric acid, particularly for feeding to a pregnant sow, as defined in the claims, e.g., in the form of a supplement, e.g. by means of addition to the drinking water. Therefore, preferably such aqueous composition comprises as chloride salt calcium chloride and as calcium binder phosphoric acid.

In an embodiment, such aqueous composition comprises between about -5 and -45 mEq/L, such as between about -10 and - 40, between about -15 and -35, between about -15 and -30, or between about -15 and -25 mEq/L of said chloride salt, such as said calcium chloride.

In an embodiment, said aqueous composition further comprises between about 0.1 and about 5 g/L, such as between 0.25 and 4.5 g/L, between 0.5 and 4 g/L, between 0.5 and 3.5 g/L, between 0.6 and 3 g/L, between 0.7 and 2.5 g/L, or between 0.75 and 2 g/L of a calcium binder, in particular of phosphoric acid.

Therefore, in a highly suitable embodiment the aqueous composition taught herein comprises between about -5 and -45 mEq/L, such as between about -10 and - 40, between about -15 and -35, between about -15 and -30, or between about -15 and -25 mEq/L of calcium chloride, and between about 0.1 and about 5 g/L, such as between 0.25 and 4.5 g/L, between 0.5 and 4 g/L, between 0.5 and 3.5 g/L, between 0.6 and 3 g/L, between 0.7 and 2.5 g/L, or between 0.75 and 2 g/L of phosphoric acid.

In an embodiment, the aqueous composition taught herein is intended for administration as drinking water to a pregnant sow In an embodiment, it is intended for administration to said animal in a period of 0 to 5 days before parturition, in particular at least in a period of 1 to 5 days before parturition. The present disclosure also provides a concentrated version of the aqueous composition taught herein, referred to as an aqueous supplement composition, which may commercially be sold for further dilution, such as intended for 1% dilution, into drinking water. In such case, the farmer will ensure dilution on-farm prior to administration thereof, resulting in the aqueous composition taught hereinabove.

The present disclosure therefore also provides an aqueous supplement composition that upon dilution in drinking water provides the aqueous composition taught hereinabove. In an embodiment, such aqueous supplement composition is suitable for 1% dilution into drinking water.

Thus, such aqueous supplement composition, when intended for 1% dilution into drinking water, may comprise between about -500 and -4500 mEq/L, such as between about -1000 and - 4000, between about -1500 and -3500, between about -1500 and - 3000, or between about -1500 and -2500 mEq/L of a chloride salt, preferably calcium chloride, and between about 10 and about 500 g/L, such as between 25 and 450 g/L, between 50 and 400 g/L, between 50 and 350 g/L, between 60 and 300 g/L, between 70 and 250 g/L, or between 75 and 200 g/L of a calcium binder, in particular phosphoric acid.

In a highly suitable embodiment, the aqueous supplement composition, when intended for 1% dilution into drinking water, comprises between about 50 and about 250 g/L calcium chloride, and between about 50 and about 250 g/L phosphoric acid.

Of course, the abovementioned amounts of chloride salt and calcium binder, in particular of calcium chloride and phosphoric acid, in such aqueous supplement composition may differ depending on the degree of dilution to be applied. The skilled person is capable of adjusting the amounts depending on the desired degree of dilution.

The compositions taught herein may further comprise at least one further ingredient such as a vitamin, a mineral, and a carrier. Further ingredients may be selected from a sugar, a stabilising agent, and a colouring agent.

The invention will now be further explained using the following non-limiting examples.

### EXAMPLES

### Example 1

In a first series of experiments, the relationship between blood pH and reproductive performance was examined. Based on these experiments it was shown that a small deviation (for example 0.03 units) from a physiological pH level may have great impact on physiological processes as established by impact on mortality, in particular stillbirth, but also on the health of piglets born alive, not only showing through a lower postnatal mortality but also by showing an increased colostrum intake. Subtle changes in pH value appear to be associated with an increase in percentage of stillborn animals and appear to affect vitality of the piglets, time they take to have their first suckle, and consequently mortality rate.

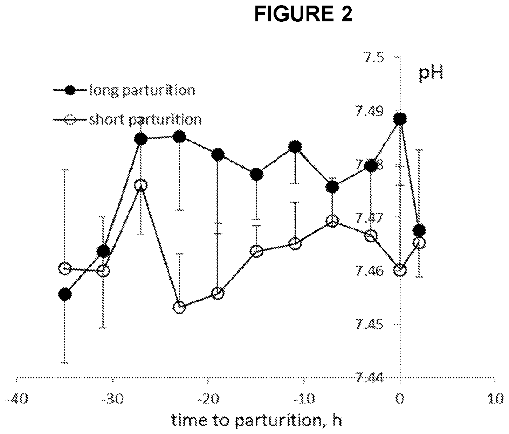

The effect of pH on stillbirth is illustrated by the fact that sows with prolonged parturition appear to have an increased pH (respiratory alkalosis) and an increased stillbirth rate. In a first experiment, 39 sows were divided in three classes of duration of parturition (14 animals had a duration of parturition > 300 minutes; 10 animals had a duration of parturition of 200-300 minutes and 15 animals had a duration of parturition of < 200 minutes). The number of stillborn animals varied from 1.6 (long duration), via 1.0 (intermediate duration) to 0.5 (short duration) while the pH varied from 7.49 (increased level) to 7.46 (physiological level) for these animals at start of parturition. The change in blood pH in sows approaching parturition is indicated in figure 2. Initially blood pH is similar, both at a physiological level of 7.46 (for these animals), however, sows that have prolonged parturition develop respiratory alkalosis (increased blood pH) in the period prior to parturition, whereas sows with normal birth show a normal (physiological) pH.

The relationship between maternal blood pH and condition of piglets is further illustrated in an experiment wherein for 94 sows, the blood pH and lactate level for piglets born to 47 sows with increased blood pH were compared to the blood pH and lactate levels of piglets born to 47 sows with pH at physiological level (see table 1).

**Table 1: pCO2 (mm Hg), pH and lactate (mmol/l) in piglets to sows with physiological and increased blood pH (>7.47)**

| Piglet blood values | born to sows with normal blood pH | born to sows with increased blood pH |
|---|---|---|
| pCO2 | 43.3 | 45.1 |
| pH | 7.41 | 7.34 |
| Lactate | 5.48 | 7.49 |

As can be seen in table 1, piglets born to sows with increased pH had a lower blood pH and higher lactate, indicating that these piglets had suffered more from oxygen insufficiency (hypoxia) during birth. This indicates that parturition for these piglets was compromised and as a consequence they had a higher stillbirth rate, and for those piglets surviving, a poorer condition at birth (clinical signs of hypoxia).

In yet another experiment it was assessed whether it would be possible to maintain the blood pH level at a physiological level by using as a pH modulator a mineral. It is known that in general blood pH can be modulated by changing the dietary electrolyte balance, or cation/anion balance of the diet. This is the difference between the total of cations and anions in the diet, and is calculated (in this experiment) as the balance between molar content of Na⁺ + K⁺ - Cl⁻ (see Block reference as mentioned here above). In the experiment a lower than normal electrolyte balance through the diet was achieved by including relatively more Cl⁻ ions in the diet than Na⁺ and K⁺ ions. A normal diet typically contains 200 to 300 mEq/kg, so at 2.5 kg intake the daily cation/anion balance would be 500 mEq/d to 750 mEq/d.

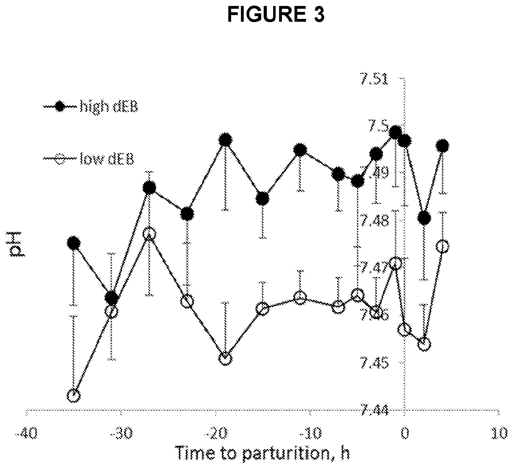

A diet was formulated to contain a cation/anion balance of 100 mEq/kg and compared to a standard diet with a cation/anion balance of 250 mEq/kg. The diets were fed from 5 days prior to farrowing at 2.7 kg per day, until the sows had farrowed, resulting in a daily cation/anion balance of 270 mEq/d and 675 mEq/d respectively. The blood pH for these sows is shown in figure 3. As can be seen, a normal (physiological) blood pH for sows on the 100 mEq/kg diet was obtained, whereas in the control group fed a regular diet the blood pH level was increased to above a physiological level prior to farrowing. In sows fed the 100 mEq/kg diet, stillbirth rate was reduced from 1.8 stillborn per litter to 0.8 stillborn piglets per litter. Litter size was 15.5 total born (alive and stillborn) piglets on average.

In another experiment, cations and anions were added to drinking water in such a balance, that the daily cation/anion balance of diet plus drinking water was reduced. For this, the drinking water contained 1.5 g calcium chloride per litre. The diets fed to the sows were the same, and hence the reduction in daily cation/anion balance was solely caused by the drinking water treatment. The cation/anion balance was reduced from 540 mEq/d in controls (normal water) to 100 to 400 mEq/d in sows with treated water; the variation in the latter was caused by variation in water intake. In this experiment, next to the calcium chloride to reduce the daily cation/anion balance, a calcium binder (phosphoric acid, PhA)) was added in a concentration of 0.01 mol/liter. The results of the study are indicated here below in table 2.

**Table 2: Effects of pH modulator and optional calcium binder on perinatal phenomena**

| | CaCI₂ + PhA* | PhA* | Control |
|---|---|---|---|
| Number of sows | 28 | 25 | 24 |
| Stillborn piglets, # | 1.0 | 1.1 | 1.6 |
| Average colostrum intake per piglet, g/24h | 447 | 408 | 422 |
| Piglets with colostrum intake < 250 g/24h | 7.1% | 15.1% | 13.9% |
| Postnatal mortality | 5.2% | 9.4% | 8.0% |

| | | | |
|---|---|---|---|
| *PhA = phosphoric acid | | | |

In this experiment, stillbirth was reduced from 1.6 to 1.0 piglets per litter. It was also confirmed that next to reducing stillbirth, the condition of piglets born alive could be improved. This is reflected in the uptake of colostrum, which was increased from 422 to 447 g/piglet (calculated on the basis of weight gain) in the first 24 h of life, even though the total number of piglets born alive was also increased from 14.1 to 14.7. More importantly, the percentage of piglets with an intake lower than 250 g colostrum, a threshold deemed critical for neonatal survival, was reduced from 14% to 7%. In addition to reducing stillbirth, the invention also decreased neonatal mortality from 8% to 5%. Remarkably, the addition of a calcium binder had no negative effect on perinatal mortality. On the contrary, given the result for the calcium binder alone on stillbirth, it is expected that the calcium binder has even increased the positive effects of the calcium chloride. It is believed that binding of dietary calcium in the gut and the inherent effect of reducing the amount of calcium available to the animal has as a consequence that the animal is triggered to mobilise calcium from its own mineral reserves in the bones. Therefore, when demand for calcium is increased as in parturition, the animal is more prepared to face this demand, and better equipped to maintain blood Ca levels. In the examples described above, blood calcium levels were indeed increased by the treatments provided (data not shown).

### Example 2

In another example, a pH blood modulator (calcium chloride) was mixed into a top-dress consisting of rice bran, which contains a calcium binding acid, namely phytic acid. The top-dress was fed from 5 days before farrowing, when the sows entered the farrowing room, until the end of parturition. The top-dress resulted in an overall electrolyte balance of the diet of 140 mEq/kg compared to 200 mEq/kg in the control group. Both groups received a total of 2.7 kg feed per sow per day. Stillbirth was reduced by 0.6 piglet per litter in the top-dress treatment (see Table 3).

**Table 3. Effect of a supplement containing a blood pH modulator and a calcium binding acid (rice bran containing phytic acid) on the outcome of parturition**

| | dEb 200 mEq/kg | dEb 140mEq/kg |
|---|---|---|
| N (number of sows) | 23 | 38 |
| Born alive | 14.1^{a} | 14.7^{b} |
| Stillborn | 1.5^{a} | 0.9^{b} |
| Sows with > 1 stillborn and/or assistance* | 71% | 44% |

| | | |
|---|---|---|
| *Manual assistance in delivery or palpation; ^{a,b}P < 0.05 | | |

### Example 3

In another trial the effect of modulating the pH in pregnant sows to physiological levels was examined, in particular the effect on pH itself and the perinatal mortality. For the trial periparturient sows were allocated to one of two treatments at 1 week before parturition. The treatments were either a high dietary electrolyte balance (250 mEq/kg) or a low dietary electrolyte balance (100 mEq/kg) by adding calcium chloride. The two diets were fed (± 2.6 kg per day) until parturition had finished. All sows received ear vein catheters at least three days before parturition to allow frequent blood sampling to measure the blood pH. Only animals with good general health based on adequate feed intake, good locomotory condition and good general visual presentation were included for assessing the results. These results are indicated in table 4.

**Table 4: Effect on stillbirth of diets differing in electrolyte balance**

| | 250 mEq/kg (high) | 100 mEq/kg (low) |
|---|---|---|
| Number of sows | 21 | 21 |
| Blood pH of the sows, 12-24 h pre-farrowing | 7.51 | 7.46 |
| Total born piglets | 15.7 | 15.4 |
| Born alive | 13.9^{a} | 14.6^{b} |
| Stillborn | 1.8^{a} | 0.8^{b} |
| Litters with 0 or 1 stillborn | 11 | 16 |

| | | |
|---|---|---|
| ^{a,b}P < 0.05 | | |

As can be seen, with the low dEB diet (i.e. 100 mEq/kg total diet), blood pH of the sows could be maintained at the physiological level, whereas with the high dEB diet, the blood pH in a period 12 to 24 hours before parturition increased to 7.51 (P < 0.01).

The effect on the total number of animals born and the number of animals born alive was not statistically significant. However, the number of stillborn piglets was decreased significantly (P = 0.09) in the sows wherein the blood pH was maintained at a physiological level. Also, the number of litters with no or at most 1 stillborn piglet was increased significantly (P = 0.07).

## Claims

1. Non-therapeutic use of an orally administered blood pH modulator which is a chloride salt, selected from the group consisting of ammonium chloride, calcium chloride and organic chloride salts, for improving colostrum production of a pregnant sow by maintaining blood pH in said pregnant sow at a physiological level which is a pH characteristic of healthy and normal functioning, wherein said physiological level is 7.45 for venous blood with a standard deviation of 0.03 within a group of animals.

2. Non-therapeutic use of a blood pH modulator according to claim 1, wherein the chloride salt is selected from the group consisting of ammonium chloride and calcium chloride.

3. Non-therapeutic use of a blood pH modulator according to any one of claims 1-2, wherein the chloride salt is calcium chloride.

4. Non-therapeutic use of a blood pH modulator according to any one of claims 1-3, wherein a calcium binder is additionally added to the diet of said pregnant sow, wherein the calcium binder is selected from the group consisting of phosphoric acid, phytic acid, a phytate, a polyphosphate, a tripolyphosphate, a phosphate and a cellulose phosphate.

5. Non-therapeutic use of a blood pH modulator according to claim 4, wherein the calcium binder is phosphoric acid or phytic acid.

6. Non-therapeutic use of a blood pH modulator according to claim 5, wherein the calcium binder is phosphoric acid.

7. Non-therapeutic use of a blood pH modulator according to any one of claims 1-6, wherein the blood pH modulator is calcium chloride and wherein a calcium binder is additionally added to the diet of said pregnant sow, wherein the calcium binder is phosphoric acid.

8. Non-therapeutic use of a blood pH modulator according to claim 7, wherein the calcium chloride and the phosphoric acid are comprised in an aqueous supplement composition intended for 1% dilution into drinking water, which aqueous supplement composition comprises between 50-250 g/L calcium chloride and between 50-250 g/L phosphoric acid.

9. Non-therapeutic use of a blood pH modulator according to any one of claims 1-8, wherein the chloride salt is administered in a period of 0 to 5 days before parturition, in particular at least in a period of 1 to 5 days before parturition.

10. Non-therapeutic use of a blood pH modulator according to any one of claims 1-9, **characterised in that** the organic chloride salt is selected from the group consisting of a betain HCI, a lysine HCI and a choline chloride.

11. Use of an aqueous composition for feeding a pregnant sow and for maintaining blood pH of said sow at a physiological level which is a pH characteristic of healthy and normal functioning which is 7.45 for venous blood with a standard deviation of 0.03 within a group of animals, comprising a blood pH modulator which is a chloride salt selected from the group consisting of ammonium chloride, calcium chloride and organic chloride salts, and a calcium binder selected from the group consisting of phosphoric acid, phytic acid, a phytate, a polyphosphate, a tripolyphosphate, a phosphate and a cellulose phosphate.

12. Use of an aqueous composition according to claim 11, wherein the chloride salt is selected from the group consisting of ammonium chloride and calcium chloride.

13. Use of an aqueous composition according to any one of claims 11-12, wherein the chloride salt is calcium chloride.

14. Use of an aqueous composition according to any one of claims 11-13, wherein the calcium binder is phosphoric acid or phytic acid.

15. Use of an aqueous composition according to any one of claims 11-14, wherein the calcium binder is phosphoric acid.

16. Use of an aqueous composition according to any one of claims 11-15, which is for administration as drinking water to a pregnant sow.

17. Use of an aqueous composition according to claim 16, which is intended for administration to said animal in a period of 0 to 5 days before parturition, in particular at least in a period of 1 to 5 days before parturition.

18. Use of an aqueous composition according to any one of claims 11-17, wherein the aqueous composition is an aqueous supplement composition suitable for 1% dilution into drinking water, said supplement composition comprising a blood pH modulator which is a chloride salt selected from a group consisting of ammonium chloride, calcium chloride and organic chloride salts, and a calcium binder selected from the group consisting of phosphoric acid, phytic acid, a phytate, a polyphosphate, a tripolyphosphate, a phosphate and a cellulose phosphate.

19. Use of an aqueous composition according to claim 18, wherein the aqueous supplement composition comprises between 50 and 250 g/L calcium chloride, and between 25 and 150 g/L phosphoric acid.

## Patentansprüche

1. Nichttherapeutische Verwendung eines oral verabreichten Blut-pH-Wert-Modulators, der ein Chloridsalz ist, das aus der Gruppe ausgewählt ist, die aus Ammoniumchlorid, Calciumchlorid und organischen Chloridsalzen besteht, zum Verbessern der Kolostrumproduktion einer trächtigen Sau durch Erhalten eines Blut-pH-Werts in der trächtigen Sau auf einem physiologischen Niveau, das ein pH-Wert ist, der für gesundes und normales Funktionieren charakteristisch ist, wobei das physiologische Niveau 7,45 für venöses Blut mit einer Standardabweichung von 0,03 innerhalb einer Gruppe von Tieren ist.

2. Nichttherapeutische Verwendung eines Blut-pH-Wert-Modulators nach Anspruch 1, wobei das Chloridsalz aus der Gruppe ausgewählt ist, die aus Ammoniumchlorid und Calciumchlorid besteht.

3. Nichttherapeutische Verwendung eines Blut-pH-Wert-Modulators nach einem der Ansprüche 1 bis 2, wobei das Chloridsalz Calciumchlorid ist.

4. Nichttherapeutische Verwendung eines Blut-pH-Wert-Modulators nach einem der Ansprüche 1 bis 3, wobei der Nahrung der trächtigen Sau ein Calciumbindemittel zusätzlich zugesetzt wird, wobei das Calciumbindemittel aus der Gruppe ausgewählt ist, die aus Phosphorsäure, Phytinsäure, einem Phytat, einem Polyphosphat, einem Tripolyphosphat, einem Phosphat und einem Cellulosephosphat besteht.

5. Nichttherapeutische Verwendung eines Blut-pH-Wert-Modulators nach Anspruch 4, wobei das Calciumbindemittel Phosphorsäure oder Phytinsäure ist.

6. Nichttherapeutische Verwendung eines Blut-pH-Wert-Modulators nach Anspruch 5, wobei das Calciumbindemittel Phosphorsäure ist.

7. Nichttherapeutische Verwendung eines Blut-pH-Wert-Modulators nach einem der Ansprüche 1 bis 6, wobei der Blut-pH-Wert-Modulator Calciumchlorid ist und wobei der Nahrung der trächtigen Sau ein Calciumbindemittel zusätzlich zugesetzt wird, wobei das Calciumbindemittel Phosphorsäure ist.

8. Nichttherapeutische Verwendung eines Blut-pH-Wert-Modulators nach Anspruch 7, wobei das Calciumchlorid und die Phosphorsäure in einer wässrigen Ergänzungszusammensetzung enthalten sind, die für eine 1%ige Verdünnung in Trinkwasser vorgesehen ist, wobei die wässrige Ergänzungszusammensetzung zwischen 50-250 g/L Calciumchlorid und zwischen 50-250 g/L Phosphorsäure umfasst.

9. Nichttherapeutische Verwendung eines Blut-pH-Wert-Modulators nach einem der Ansprüche 1 bis 8, wobei das Chloridsalz in einem Zeitraum von 0 bis 5 Tagen vor einer Geburt verabreicht wird, insbesondere mindestens in einem Zeitraum von 1 bis 5 Tagen vor der Geburt.

10. Nichttherapeutische Verwendung eines Blut-pH-Wert-Modulators nach einem der Ansprüche 1 bis 9, **dadurch gekennzeichnet, dass** das organische Chloridsalz aus der Gruppe ausgewählt ist, die aus einem Betain-HCI, einem Lysin-HCI und einem Cholinchlorid besteht.

11. Verwendung einer wässrigen Zusammensetzung zum Füttern einer trächtigen Sau und zum Erhalten des Blut-pH-Werts der Sau auf einem physiologischen Niveau, das ein pH-Wert ist, der für gesundes und normales Funktionieren charakteristisch ist, der 7,45 für venöses Blut mit einer Standardabweichung von 0,03 innerhalb einer Gruppe von Tieren ist, umfassend einen Blut-pH-Wert-Modulator, der ein Chloridsalz ist, das aus der Gruppe ausgewählt ist, die aus Ammoniumchlorid, Calciumchlorid und organischen Chloridsalzen ausgewählt ist, und ein Calciumbindemittel, das aus der Gruppe ausgewählt ist, die aus Phosphorsäure, Phytinsäure, einem Phytat, einem Polyphosphat, einem Tripolyphosphat, einem Phosphat und einem Cellulosephosphat besteht.

12. Verwendung einer wässrigen Zusammensetzung nach Anspruch 11, wobei das Chloridsalz aus der Gruppe ausgewählt ist, die aus Ammoniumchlorid und Calciumchlorid besteht.

13. Verwendung einer wässrigen Zusammensetzung nach einem der Ansprüche 11 bis 12, wobei das Chloridsalz Calciumchlorid ist.

14. Verwendung einer wässrigen Zusammensetzung nach einem der Ansprüche 11 bis 13, wobei das Calciumbindemittel Phosphorsäure oder Phytinsäure ist.

15. Verwendung einer wässrigen Zusammensetzung nach einem der Ansprüche 11 bis 14, wobei das Calciumbindemittel Phosphorsäure ist.

16. Verwendung einer wässrigen Zusammensetzung nach einem der Ansprüche 11 bis 15, die für eine Verabreichung als Trinkwasser an eine trächtige Sau ist.

17. Verwendung einer wässrigen Zusammensetzung nach Anspruch 16, die für die Verabreichung an das Tier in einem Zeitraum von 0 bis 5 Tagen vor der Geburt, insbesondere mindestens in einem Zeitraum von 1 bis 5 Tagen vor der Geburt vorgesehen ist.

18. Verwendung einer wässrigen Zusammensetzung nach einem der Ansprüche 11 bis 17, wobei die wässrige Zusammensetzung eine wässrige Ergänzungszusammensetzung ist, die für die 1%ige Verdünnung in Trinkwasser geeignet ist, die Ergänzungszusammensetzung umfassend einen Blut-pH-Wert-Modulator, der ein Chloridsalz ist, das aus einer Gruppe ausgewählt ist, die aus Ammoniumchlorid, Calciumchlorid und organischen Chloridsalzen besteht, und ein Calciumbindemittel, das aus der Gruppe ausgewählt ist, die aus Phosphorsäure, Phytinsäure, einem Phytat, einem Polyphosphat, einem Tripolyphosphat, einem Phosphat und einem Cellulosephosphat besteht.

19. Verwendung einer wässrigen Zusammensetzung nach Anspruch 18, wobei die wässrige Ergänzungszusammensetzung zwischen 50 und 250 g/L Calciumchlorid und zwischen 25 und 150 g/L Phosphorsäure umfasst.

## Revendications

1. Utilisation non thérapeutique d'un modulateur de pH sanguin administré par voie orale, qui est un sel de chlorure choisi dans le groupe constitué de chlorure d'ammonium, de chlorure de calcium et de sels de chlorures organiques, pour améliorer la production de colostrum par une truie enceinte en maintenant le pH sanguin de ladite truie à un niveau physiologique qui est une caractéristique de pH d'un fonctionnement normal et sain, dans laquelle ledit niveau physiologique est 7,45 pour du sang veineux avec une déviation standard de 0,03 au sein d'un groupe d'animaux.

2. Utilisation non thérapeutique d'un modulateur de pH sanguin selon la revendication 1, dans laquelle le sel de chlorure est choisi dans le groupe constitué de chlorure d'ammonium et de chlorure de calcium.

3. Utilisation non thérapeutique d'un modulateur de pH sanguin selon l'une quelconque des revendications 1 à 2, dans laquelle le sel de chlorure est du chlorure de calcium.

4. Utilisation non thérapeutique d'un modulateur de pH sanguin selon l'une quelconque des revendications 1 à 3, dans laquelle un liant calcique est en outre ajouté au régime alimentaire de ladite truie enceinte, dans laquelle le liant calcique est choisi dans le groupe constitué d'acide phosphorique, d'acide phytique, d'un phytate, d'un polyphosphate, d'un tripolyphosphate, d'un phosphate et d'un phosphate de cellulose.

5. Utilisation non thérapeutique d'un modulateur de pH sanguin selon la revendication 4, dans laquelle le liant calcique est de l'acide phosphorique ou de l'acide phytique.

6. Utilisation non thérapeutique d'un modulateur de pH sanguin selon la revendication 5, dans laquelle le liant calcique est de l'acide phosphorique.

7. Utilisation non thérapeutique d'un modulateur de pH sanguin selon l'une quelconque des revendications 1 à 6, dans laquelle le modulateur de pH sanguin est du chlorure de calcium et dans laquelle un liant calcique est en outre ajouté au régime alimentaire de ladite truie enceinte, dans laquelle le liant calcique est de l'acide phosphorique.

8. Utilisation non thérapeutique d'un modulateur de pH sanguin selon la revendication 7, dans laquelle le chlorure de calcium et l'acide phosphorique sont compris dans une composition de supplément aqueux prévue pour une dilution de 1% dans de l'eau de boisson, laquelle composition de supplément aqueux comprend entre 50 et 250 g/L de chlorure de calcium et entre 50 et 250 g/L d'acide phosphorique.

9. Utilisation non thérapeutique d'un modulateur de pH sanguin selon l'une quelconque des revendications 1 à 8, dans laquelle le sel de chlorure est administré dans une période allant de 0 à 5 jours avant parturition, en particulier au moins dans une période allant de 1 à 5 jours avant parturition.

10. Utilisation non thérapeutique d'un modulateur de pH sanguin selon l'une quelconque des revendications 1 à 9, **caractérisée en ce que** le sel de chlorure organique est choisi dans le groupe constitué d'une bétaïne HCI, d'une lysine HCI et d'un chlorure de choline.

11. Utilisation d'une composition aqueuse pour nourrir une truie enceinte et pour maintenir le pH sanguin de ladite truie à un niveau physiologique qui est une caractéristique de pH d'un fonctionnement normal et sain qui est de 7,45 pour du sang veineux avec une déviation standard de 0,03 au sein d'un groupe d'animaux, comprenant un modulateur de pH sanguin qui est un sel de chlorure choisi dans le groupe constitué de chlorure d'ammonium, de chlorure de calcium et de sels de chlorures organiques, et un liant calcique choisi dans le groupe constitué d'acide phosphorique, d'acide phytique, d'un phytate, d'un polyphosphate, d'un tripolyphosphate, d'un phosphate et d'un phosphate de cellulose.

12. Utilisation d'une composition aqueuse selon la revendication 11, dans laquelle le sel de chlorure est choisi dans le groupe constitué de chlorure d'ammonium et de chlorure de calcium.

13. Utilisation d'une composition aqueuse selon l'une quelconque des revendications 11 à 12, dans laquelle le sel de chlorure est du chlorure de calcium.

14. Utilisation d'une composition aqueuse selon l'une quelconque des revendication 11 à 13, dans laquelle le liant calcique est de l'acide phosphorique ou de l'acide phytique.

15. Utilisation d'une composition aqueuse selon l'une quelconque des revendication 11 à 14, dans laquelle le liant calcique est de l'acide phosphorique.

16. Utilisation d'une composition aqueuse selon l'une quelconque des revendication 11 à 15, destinée à être administrée à une truite enceinte en tant qu'eau de boisson.

17. Utilisation d'une composition aqueuse selon la revendication 16, destinée à être administré audit animal dans une période allant de 0 à 5 jours avant parturition, en particulier au moins dans une période allant de 1 à 5 jours avant parturition.

18. Utilisation d'une composition aqueuse selon l'une quelconque des revendication 11 à 17, dans laquelle la composition aqueuse est une composition de supplément aqueux adapté à une dilution à 1% dans de l'eau de boisson, ladite composition de supplément comprenant un modulateur de pH sanguin qui est un sel de chlorure choisi dans le groupe constitué de chlorure d'ammonium, de chlorure de calcium et de sels de chlorures organiques, et un liant calcique choisi dans le groupe constitué d'acide phosphorique, d'acide phytique, d'un phytate, d'un polyphosphate, d'un tripolyphosphate, d'un phosphate et d'un phosphate de cellulose.

19. Utilisation d'une composition aqueuse selon la revendication 18, dans laquelle la composition de supplément aqueux comprend entre 50 et 250 g/L de chlorure de calcium et entre 25 et 150 g/L d'acide phosphorique.
